(19) Europäisches Patentamt
European Patent Office
Office européen des brevets

(11) Publication number: **0 087 397**
**A2**

# (12) EUROPEAN PATENT APPLICATION

(21) Application number: 83830031.7

(22) Date of filing: 09.02.83

(51) Int. Cl.³: C 07 C 149/23
A 61 K 31/16
//C07C153/11, C07C149/20

(30) Priority: 12.02.82 IT 1965282

(43) Date of publication of application:
31.08.83 Bulletin 83/35

(84) Designated Contracting States:
AT BE CH DE FR GB LI LU NL SE

(71) Applicant: Farmatis S.r.l.
Corso Europa n. 5
I-20100 Milano 1(IT)

(72) Inventor: Scalesciani, Juan Boris Aldo
Calle J.B. Alberdi
Buenos Aires(AR)

(74) Representative: Gervasi, Gemma et al,
Studio Brevetti e Marchi NOTARBARTOLO & GERVASI
33, Viale Bianca Maria
I-20122 Milano(IT)

## (54) Propionylglycine derivative, process for its preparation and therapeutic compositions which contain it as the active principle.

(57) The calcium salt from beta-mercapto-propionylamidoacetic acid was prepared having the following formula:

O

$CH_2 - CH_2 - CO - NH - CH_2 - C$ ½ $Ca^+$

O

The new compound has revealed a series of characteristics which make it a pharmaceutical product of the mucolytic type that is far superior to anu of the thiolic mucolytic products known today.

EP 0 087 397 A2

"Propionylglycine derivative, process for its preparation and therapeutic compositions which contain it as the active principle".

This invention concerns the calcium salt of beta-mercapto propionylamidoacetic acid having the formula:

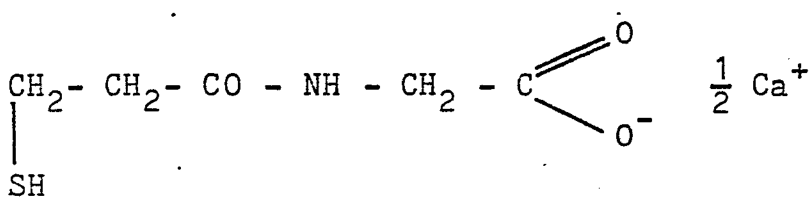 (I)

and the use of this product in human therapy as a muco lytic.

It is known that the liquefying of excreta by chemical means constitutes a fundamental therapeutic objective in modern therapy regarding obstructive, chronic bronchopneumo phatics.

Since the bronchial secretion generally consists of purulent exudates and mucous - the exudates being DNA - rich and the mucous mucoprotein-rich, the chemical products used up to now as liquefiers have been enzymes, surfactants and sulfurated compounds.

The enzymes and surfactants are not consistent in their effectiveness and are not well tolerated, giving rise to allergies, bronchial asthma attacks and irritation of the

mucous membranes:
The thiolitic compounds, on the other hand, are much more interesting, especially certain derivatives of L-cysteine. In actual practice, however, these products have been shown to have certain negative aspects which limited their range of use.
The compound which has given to date the best results has been N-acetylcysteine.
We have recently found that the calcium salt from beta--mercaptopropionylamidoacetic acid - which forms the subject of this invention and whose formula, (I), is given on page 1 - is not only decidedly, and unexpectedly, superior to N-acetylcysteine in mucolytic activity, but also demonstrates having a series of additional characteristics which make this pharmaceutical product of exceptional interest in the field of mucolytics.
The new product explained in this invention is prepared by salting out beta-mercaptopropionylamidoacetic with a calcium base in an aqueous solution.
Beta-mercaptopropionylamidoacetic acid can be prepared by various processes.
These processes are described below:
a) Made by reacting glycine with a bromo-propionyl halogenide and reacting this obtained product with thiobenzoic acid.
The beta-benzoylmercaptopropionylamidoacetic acid is separated and hydrolized to beta-mercaptopropionylamidoacetic acid in an alkaline environment.
The steps of this process are shown below and the operative details are given in Example 1, which appears in the examples which follow:

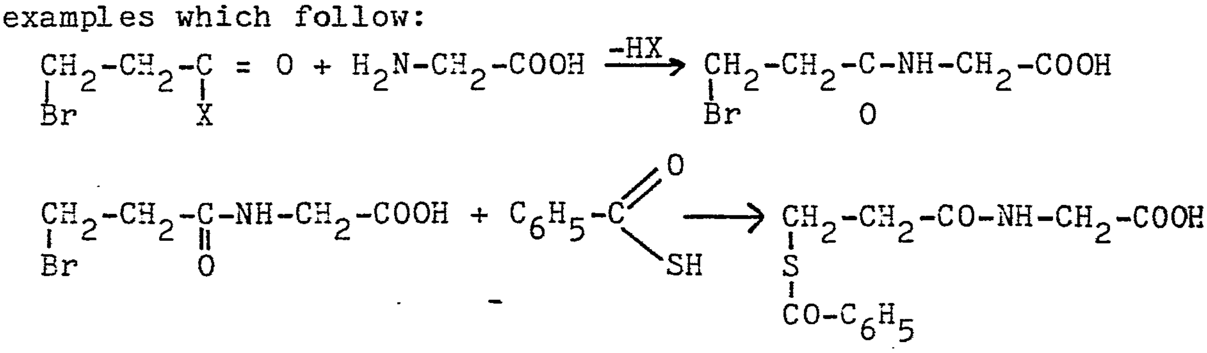

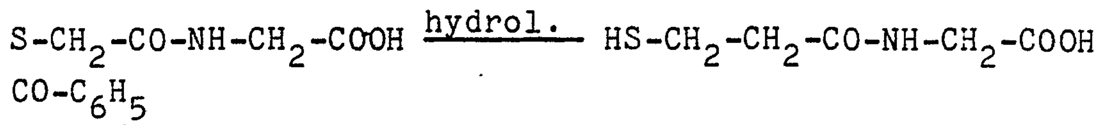


b) Made by reacting propiothiolactone with the ethylic ester of glycine. The thus obtained ethylic ester of beta-mercaptopropionylamidoacetic acid is hydrolized to beta-mercaptopropionylamidoacetic acid in alkaline environment.

The process is represented by the following reactions:

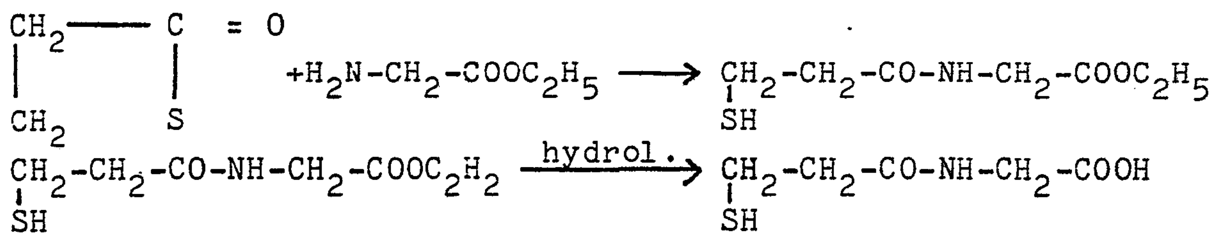


The operative details are given in Example 2.

c) By oxidation with $H_2O_2$ of beta-mercapto propionic acid, the $\beta,\beta'$-dithiopropionic acid is obtained. The latter is changed into the corresponding dihalide which, by reaction with glycine, leads to $\beta,\beta'$-dithiopropionamidoacetic acid. By electrochemically reducing the last named disulphide, -mercaptopropionylamidoacetic acid is obtained.

The process is represented by the following reaction scheme:

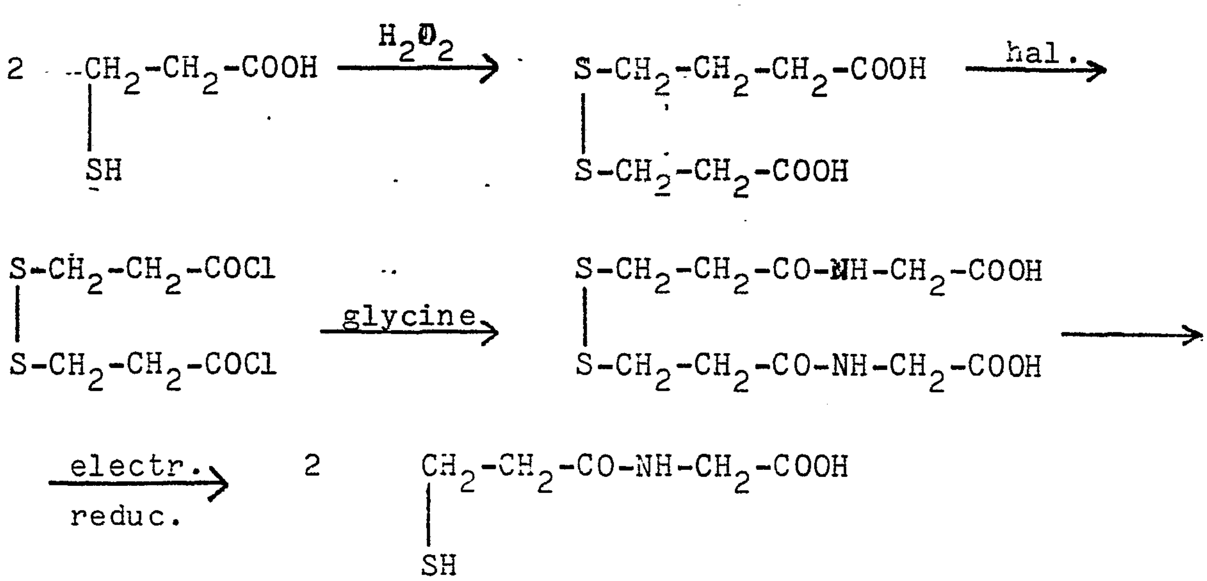


The operative details are given in Example 3.

First of all, the obtained beta-mercaptopropionylamidoacetic acid, as described in any of these processes, is treated with either a hydrate or carbonate of calcium at a temperature of between 20 and 50°C.

EXAMPLE 1

Beta-benzoylmercaptopropionylamidoacetic Acid

75 g of glycine are dissolved in a 500 ml, 2M aqueous solution of NaOH. The solution is cooled to 0-5°C. While agitating vigorously, 550 ml of 2M aqueous NaOH and 180 g of beta-bromopropionylchloride in three portions and in order. The reaction mixture is then agitated at room temperature for four hours. A mixture of 150 g of thiobenzoic acid and 100 g of potassium carbonate in one liter of water is added and the reaction is allowed to take place at room temperature and for an overnight period. Acidification is done with conc. HCL and the obtained solids are filtered out, washed with cold water and crystallized with methanol and water. 240 g of beta-benzoylmercaptopropionylamidoacetic acid are obtained having a m.p. of 130°C.

Beta-mercaptopropionylamidoacetic Acid

26.7 g of beta-benzoylmercaptopropionylamidoacetic acid are dissolved in a mixture of 100 ml of water and 65 ml of conc. $NH_4OH$. Agitation is carried out for four hours at ambient temperature, after which the reaction mixture is diluted with water and filtration is performed. The filtrate is extracted with ethyl acetate, the aqueous phase is acidified with conc. HCL and, after saturating with sodium chloride, two extractions are made with ethyl acetate. The organic extracts are washed with a saturated sodium chloride solution, dried on

magnesium sulfate and dry-concentrated. The product is crystallized with ethyl acetate. 13 g of beta-mercaptopropionylamidoacetic acid are thus obtained having a m.p. of 99-101°C.

EXAMPLE 2

Beta-mercaptopropionylamidoacetic Acid Ethylic Ester

17.6 g of propiothiolactone are added to a solution of 20 g of ethylic ester of glycine in 100 ml of anhydrous tetrahydrofurane cooled in an ice bath. The reaction mixture is agitated for 10 minutes in the ice bath and for three hours at ambient temperature. The mixture is then diluted with ethyl acetate and washed, first, with 5% aqueous solution of $KHSO_4$ and, then, with water. The organic phase is dried on magnesium sulfate and dry-concentrated under a vacuum. The residue is crystallized with ethanol and water. 28 g of beta-mercaptopropionylamidoacetic ethylic ester acid are obtained having a m.p. of 69-71°C.

Beta-mercaptopropionylamidacetic Acid

100 ml of 1M aqueous NaOH are added to a solution of 19.1 g of beta-mercaptopropionylamidoacetic acid ethylic ester and 200 ml of dioxane. The mixture is agitated for two hours at ambient temperature. The organic solvent is separated under a vacuum, dilution is done using water and ethyl acetate is used for the extraction. The aqueous phase is acidified with conc. HCL, saturated with sodium chloride and two extractions are made using ethyl acetate. After washing with a saturated sodium chloride solution, the organic phase is dried on magnesium sulfate and dry-concentrated. Crystallization using ethyl acetate produces the final product of 12.5 g of beta-mercaptopropionylamidoacetic acid.

EXAMPLE 3

Beta, Beta'-dithio-dipropionic Acid

9.6 ml of 36% $H_2O_2$ are added to a solution of 21.2 g of beta-mercaptopropionic acid and 200 ml of water. The solution is put in the agitator and after about an hour a white precipitate begins to form and increases as time goes on. After agitating overnight, the product is filtered out and a second quantity is obtained from the mother liquor, to be added to the first, followed by drying under vacuum. 20 grams are obtained, and the product has a m.p. of 156°C.

Beta, Beta'-dithio-dipropionylamidoacetic Acid

42 g of beta, beta'-dithio-dipropionic acid are treated with 80 ml of thionyl chloride refluxing for two hours. After cooling, the excess thionyl chloride is eliminated under a vacuum, which produces an oily residue that goes directly to the glycine condensation operation.

15 g of glycine are dissolved in 100 ml of 2M aqueous NaOH and the solution is cooled to 0-5°C. The solution is put under vigorous agitation and the chloride of the previously-obtained acid and 100 ml of 2M NaOH are added, a little at a time and in alternating sequence. The reaction mixture is then agitated at ambient temperature for four hours. It is then acidified with cond. HCL. After several hours a white precipitate forms which is filtered out, washed with water and dried under a vacuum. After crystallizing, using water, 20 grams of product are obtained which have a m.p. of 196-198°C.

Beta-mercaptopropionylamidoacetic Acid

10 g of beta, beta'-dithio-dipropionylamidoacetic acid and 5 g of sodium bicarbonate are dissolved in 400 ml of water.

The solution is subjected to electrolysis, using a mercury electrode polarized at -1.4 volts. When the electrochemical reduction is completed, the solution is acidified with conc. HCL and saturated with sodium chloride. Two extractions are made using ethyl acetate. The extracted organic material is put together and dried on magnesium sulfate, followed by filtration and dry-concentration. 8 grams of beta-mercaptopropionylamidoacetic acid are obtained having a m.p. of 100-102°C.

EXAMPLE 4

16.3 g of beta-mercaptopropionylamidoacetic are put in solution with 50 ml of water. The solution is then agitated while 5.5 g of calcium carbonate are added. The temperature of the mixture is brought to 50°C and the suspension kept under agitation for one hour. The excess calcium carbonate is filtered out. The filtrate is put under a vacuum for concentrating, keeping the temperature at a constant 50°C, until a syrupy consistency is obtained. The oil is allowed to drop slowly into 200 ml of absolute ethanol as a very fine thread. The suspension is kept under brisk agitation until a white solid material crystallizes out completely. This material is filtered out and dried, under a vacuum, on sulfuric acid. 17.8 g of product are obtained with almost quantitative efficiency.

Elemental analysis of product with MW = 182.2

| | | | |
|---|---|---|---|
| As Calculated for $C_5H_8NO_3SCa_{\frac{1}{2}}$ : | 32.9% C | 4.4% H | 7.7% N |
| As Found : | 33.0% C | 4.5% H | 7.7% N |

The pharmacological activity of the new product was determined, in comparison with N-acetyl-cysteine, the correspond-

free acid (beta-mercaptopropionylamidoacetic acid), alpha-mercaptopropionylamidoacetic acid and their alkaline salts. As mentioned, N-acetyl-cysteine is a the thiolic derivative which has to date been the most used for human therapy. The latter are the thiolic compounds having mucolytic activity which have the closest correlation from the point of view of chemical structure.

I - Pharmacodynamics

a) "In vitro" Tests

- Mucolytic activity on gastric mucin.

The mucolytic activity was measured using DIFCO gastric mucin (10 g dissolved in 100 ml of physiological solution). The mucolytic compounds under test were incubated for 5 minutes at 25°C under constant agitation, mixing 1 ml of aqueous solution of product, having increasing molar quantities of the product being considered, with 10 ml of mucin. The viscosity was measured both before and after incubation, using an Ostwald viscosimeter with 1.5 mm capillaries. Table 1 shows post-incubation viscosity values as residual percentage of the initial viscosity. Each value represents the average of six readings.

TABLE 1

Mucolytic activity on gastric mucin.

| Molar | Residual Viscosity in % | | |
|---|---|---|---|
| Conc. | AC | MP | MPCa |
| 0.015 | 91 | 88 | 83 |
| 0.03 | 86 | 84 | 75 |
| 0.06 | 66 | 65 | 59 |

AC = N-acetylcysteina (used in solution tamponed with

bicarbonate of soda)

MP = beta-mercaptopropionylamidoacetic acid (in solution tamponed with bicarbonate of soda)

MPCa = compound I

- Mucolytic activity on nonpurulent excreta

The mucolytic activity was measured using a pool of nonpurulent excreta obtained from chronic bronchitis patients. The excreta was diluted 1:1 with a physiological solution and homogenized for three minutes - using a glass homogenizer - and then filtered through gauze and used immediately. The method used here is the same as described in the previous paragraph. In this case, however, the residual viscosity was determined after 5 minutes at 25°C (a) and after 40 minutes at 37°C (b). It was found that with mucin the liquefying effect of the compounds reached a maximum after only 5 minutes, whereas with the excreta the liquefying effect reached a maximum after 40 minutes.

TABLE 2

Mucolytic activity on excreta.

| Molar Conc. | Residual Viscosity in % | | | | | |
|---|---|---|---|---|---|---|
| | AC | | MP | | MPCa | |
| | (a) | (b) | (a) | (b) | (a) | (b) |
| 0.015 | 87 | 82 | 82 | 78 | 76 | 71 |
| 0.03 | 68 | 62 | 63 | 56 | 44 | 36 |
| 0.06 | 40 | 33 | 40 | 31 | 32 | 24 |

AC and MP in tamponed solution.

The data shown in Tables 1 and 2 make it clear that the three thiolic compounds have a direct mucolytic effect on both mucin and excreta with the activity being proportional to the molar concentration in accordance with a linear dosage-effect regression which is highly significant. The differences, however, are only substantial when the MPCa is compared with the other two compounds, AC and MP. The activity of these two compounds are seen to be practically equivalent.

When the tests were repeated using alpha-mercaptopropionylamidoacetic acid, MP/A, and S-carboxymetylcystine, CM, the values obtained with the MP/A compound were substantially the same as those obtained with the beta isomer (MP). The CM compound, on the other hand, gave values that were 30% lower than those obtained with the MP compound.

What with the considerable structural affinity of all the compounds considered and the presence of the -SH group in all of them, it is very surprizing to see that the new compound, (I), has a decidedly higher mucolytic activity, which is to be attributed to the unforeseeable sinergic action of the Ca ion.

b) - "<u>In vivo</u>" <u>Tests</u>

- Mucolytic activity

This was determined by means of bronchial hypersecretion induced in rats using $SO_2$. The rats were treated by oral administration of 0.25 g/kg/die of mucolytic product for a period of two weeks, followed by inhalation of 300 ppm of $SO_2$ for two hours per day for a total of 90 hours. Table 3 below shows the number of rats which developed bronchial obstruction, expressed as a percentage of the animals not protected.

TABLE 3

| Compound | % |
|---|---|
| Control | 80 |
| MP | 16 |
| MP/A | 14.5 |
| AC | 16 |
| CM | 29 |
| MPCa | 12.5 |

It is evident that the mucolytic action "in vivo" of the new product (I) is clearly superior than that of the known mucolytics.

Histological and histochemical (P.A.S. coloration) tests, furthermore, showed that the bronchial mucosa of the rats treated with the new compound had a significant reduction of calyciform hyperplasia (40% less than those of the control group) and of the number of muciparous cells, with the almost complete persistence of the -SH groups (D.D.D. coloration) in the cells of the bronchial mucosa.

The -SH groups in the control animals were reduced by 70%. These results can be correlated to a cytotrophic-protective action.

- Trophic-Protective Activity

This was determined in rats by the administration of 0.25 g/kg/die, orally, of the product for a period of ten days while, contemporaneously, treating with nebulized $CCl_4$ (5 applications of 2 minutes/die).

The new compound prevented intrabronchial desquamation and reactive hyperplasia of the mucosa to the extent of 65%, while protection was only a maximum of 50% under the same

conditions and using the MP, MP/A, AC and CM compounds. The new compound, therefore, has a superior trophic-protective action in comparison to the known products.

- Antiphlogistic Activity

This was determined on rats by means of the administration of the product, using an esophagus probe, one hour before making a subcutaneous injection of 0.05 ml of 1% carragenin solution in the right hind paw. After three hours, the volume of the treated paw was compared to the left hind paw, using a plethysmograph.

The following table, Table 4, shows the obtained $DE_{50}$ values.

TABLE 4

Antiphlogistic Activity

| Compound | $DE_{50}$ (mg/kg) |
|---|---|
| MPCa | 650 |
| MP | 785 |
| MP/A | 780 |
| AC | 830 |
| CM | not measurable |
| ASA | 100 |

ASA = acetylsalicylic acid

The antiphlogistic activity of the new compound is clearly greater than the other strictly correlated mucolytics and indicates its capacity for inhibiting plasmatic quinines and thereby providing the reduction of bronchospasm in hypersecretive and obstructive bronchopneumonopathics.

- Antibronchospasmic Activity

This was evaluated by treating the test animals with 0.1 g/kg i.p. of the product and then subjecting them to aerosol hista-

minic shock (histamine phosphate at approx. 140 mm Hg) after two hours following the pretreatment. The time was taken for the onset of the convulsions after having started administering the aerosol.

The times obtained are shown as follows:

| | |
|---|---|
| control | 120 sec. |
| MPCa | 250 sec. |
| MP | 210 sec. |
| MP/a | 200 sec. |
| AC | 210 sec. |

There was an increase of 30% more time,48 hours after having administered the MPCa pharmaceutical product, while protective action for the other products was reduced to practically zero.

These tests definitely prove that the new compound (I) provides a more intense and protective action against the experimentally induced bronchospasm.

In conclusion, the series of "in vivo" tests show that the new product has a mucolytic activity, is cytotrophic-protective for bronchial mucosa, is anti-inflammatory and antibronchospasmic to a superior degree than are the known mucolytic products which are structurally isomeric or structurally very closely analogous.

Also in this case, this greater activity was in no way predictable and can only be explained by the unexpected sinergic action of the Ca ion.

II - Toxicology

a) - <u>Acute Toxicity</u>

The acute toxicity determined in mice is shown by the values

in Table 5 below:

TABLE 5

| Compound | MPCa | MP | MP/A | AC |
|---|---|---|---|---|
| $DL_{50}$ g/kg | | | | |
| i.p | 1.71 | 1.54 | 1.28 | 0.40 |
| e.v. | 3.47 | 3.10 | 2.24 | - |
| os | 3.51 | 3.20 | 2.19 | - |

The determination of the i.p., e.v. and os acute toxicity values for the rats did not provide statistically significant differences as compared to the determination for the mice.

The acute toxicity of the new product is lower than that of the products that were compared.

b) - Subacute, chronic and fetal toxicity

The subacute toxicity of the new product being practically the same for either case of oral or endovenous administration, the administration was only done by means of a gastric probe.

The subacute toxicity tests were carried out for 12 weeks on the rats, the chronic toxicity on rats and dogs for 6 months (0.1 - 0.5 g/kg/die) and the fetal toxicity tests were carried out on rats (0.1 - 0.5 g/kg/die between the 6th and 16th days gravidity) and on rabbits (0.1 - 0.3 g/kg/die between the 6th and 18th days of gravidity). Very good tolerability for the new product was demonstrated.

In fact, no significant variations were observed in the evolution of the body weight, nor in the weight or macro or microscopic aspect of the main organs, nor in the hemochromocytometric tests, nor in the main hepatic functional tests

(bilirubin, SGOT, SGPT, alkaline phosphatase), nor as regards tests for uricemia, cholesterolemia, glycemia, azotemia and the standard urine tests. The treatment, furthermore, had no negative effects on the mothers, nor on the characteristics of the offspring as regards the number born, fetal losses and weights of offspring and fetuses, nor as regards embryonic and fetal development, on the basis of the number of malformations, variations or anomalies involving the skeleton or viscera.

In order to evaluate the tolerability at the lung level, for the same rats undergoing the chronic toxicity test, a determination of the influence made at the surfactant level was done at the termination of the treatment. A phospholipids analysis was made, obtaining qualitative data by means of TLC chromographic analysis and quantitative data by phosphorous determination. Gas chromographic analysis was made of the fatty acid content in liposomes separated by centrifuging from the liquid in which the excised lungs had been washed. No significant differences were observed as compared with the control group. Likewise, no significant differences were observed, as compared to the control group, regarding the amount of type II pneumocytes and their granular content (fixed and colored with Os-I).

III - Pharmcokinetics

The pharmcokinetic tests were carried out on rats after having administered 0.8 g/kg of the new product, orally, and using an esophagus probe.

The urinary elimination tests were carried out using the titrimetric method. Each sample was treated with an equal volume of 20% HCL, titrating the sulfhydrylic groups with M/100 potassium bromate until the disappearance of the yellow color and calculating the amount of contained new compound (I) by means of a calibrated curve obtained by the same method. The velocity of urinary excretion is maximum within the first two hours after administration. Maximum values obtained after 5 hours amount to about 20% of the amount administered. Administering the same dose (0.8 g/kg) to a rat by means of a rectal suppository of very small dimensions, the cumulative amount in per cent eliminated in the urine within the first five hours is about 12 %.

These results give direct proof that the new compound is absorbed well, orally and rectally, and indirectly show its metabolic utilization, in analogy with numerous compounds having a free thiolic function (derivatives of cysteine, cysteamine, glutathione and thiopronine).

IV - Clinical Tests

In the clinical field, preliminary tests were performed with the new compound by giving daily doses of 0.5 g, parenterally, 0.8 g, by aerosol, and 1 g, either orally or rectally, to chronic bronchitis cases. There was a progressive diminuition of excreta viscosity at the 4th-5th day of treatment, 70% in the case of purulent expectoration and 50% in the case of mucopurulent expectoration. The values were measured by means of a cone and plate, rotary viscosimeter. There was an average increase of 20% in vital capacity, 15% in VEMS and a reduction of residual volume of about 20%.

It was particularly noted that the kallikrein did not produce any quinic action on the bronchial tone with the aerosol treatment. In other words, no bronchospasmic manifestations were observed (evaluation with plethysmograph in broncholabile patients). This test proves once more that the new product is well tolerated in comparison with the other compounds having free thiolic action.

Compared to the corresponding free acid tamponed with NaOH, the new compound, furthermore, is characterized as having a more marked antiphlogistic and antibronchospasmic liquefying effect, which is certainly attributable to an unforeseen sinergic action provided by the calcium ion. There is a stronger detergent effect on the mucosa of the bronchi and better local tolerability with the aerosol (less irritation of the air passages). Tolerability is also better with intramuscular injections (less local soreness), with pills and/or capsules and/or granules (greater gastric tolerance and and less nauseous eructation) and with suppositories (less anal-rectal irritation).

Also clinically, therefore, the new compound - due to the presence of the calcium ion - proves to be decidedly better, both as regards its therapeutic activity and its local tolerability, not only with respect to the corresponding free acid (MP) but also with respect to the well-known alpha isomeric acid (MP/A) and the structural isomer N-acetylcysteine (AC).

By way of example, we are listing below a few typical formulations for this compound specified by this invention. (The doses given are for adults. Children require only half these doses.)

- <u>Vials or small flasks for aerosol</u>

Beta-mercaptopropionylamidoacetate of calcium, 250 or 400 mg.

Sterile q.o. distilled water, 3 ml.

S. 1-2 vials or small flasks per day by means of aerosol, nebulization, washing or instillation.

- Vials for parenteral administration

Beta-mercaptopropionylamidoacetate of calcium, 250 mg.

Sterile, bidistilled water for preparing injection, q.b., 3 ml.

S. 1-2 vials per day by deep i.m. injection or slow e.v. injection.

- Pills or capsules

Beta-mercaptopropionylamidoacetate of calcium, 250 mg.

Starch and lactose q.b., 0.4 g

S. 2-4 pills or capsules daily

- In granular form for oral use

Beta-mercaptopropionylamidoacetate of calcium, 250 mg

Excipients (anhydrous grapefruit juice 10%, essential citrus oils 0.2%, citric acid 5%, saccharose 84.8%) q.b., 6 g.

S. 2-4 small packets in half a glass of water per day.

- Suppositories

Beta-mercaptopropionylamidoacetate of calcium, 500 mg

Fatty vegetable acid glycerides q.b., 3 g

S. 1-2 suppositories per day

Claims

1. Beta-mercaptopropionylamidoacetate of calcium having the following formula :

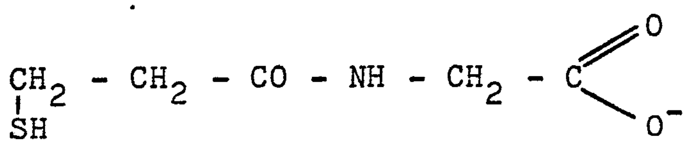 $\frac{1}{2}$ $Ca^+$ (I)

2. The process for the preparation of beta-mercaptopropionylamidoacetate of calcium of formula (I) is characterized by the fact that beta-mercaptopropionylamidoacetic acid is made to react with a Ca base, in an aqueous solution, at a temperature of from 20° to 50°C.

3. The process, according to claim 2, whereby beta-mercapto propionylamidoacetic acid is prepared from a bromopropylhalogenide and glycine by reacting their condensation product with thiobenzoic acid and alkaline hydrolysing of the beta-benzoulmercaptopropionamidoacetic acid thus obtained.

4. The process, according to claim 2, in which beta-mercaptopropionylamidoacetic acid is prepared from propiothiolactone and ethylic ester of glycine and by alkaline hydrolysis of the ethylic ester of the beta-mercaptopropionylamidoacetic acid thus obtained.

5. The process, according to claim 2, whereby beta-mercapto propionylamidoacetic acid is prepared from beta-mercaptopropionic acid by oxidixing with $H_2O_2$; beta, beta'-dithiopropionic acid is transformed into the corresponding dihalogenide; reacting this product with glycine is then done followed by the electrochemical reduction of the beta, beta'-dithiodipropionamidoacetic acid thus obtained.

6. Therapeutic and mucolytically active ingredients contained as active principle of beta-mercaptopropionylamidoacetate

of calcium.

7. The use of beta-mercaptopropionylamidoacetate of Ca as a mucolytic agent in human therapy.

0087397

USTRIAN CLAIMS

1. The process for the preparation of beta-mercaptopropionylamidoacetate of calcium of formula

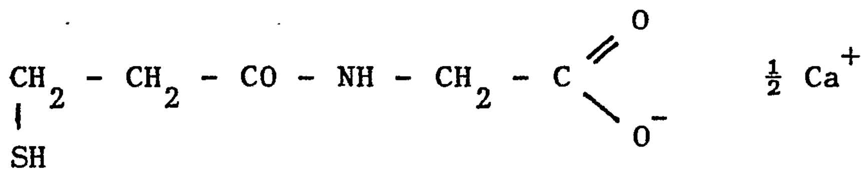

(I)

is characterized by the fact that beta-mercaptopropionylamidoacetic acid is made to react with a Ca base, in an aqueous solution, at a temperature of from 20° to 50°C.

2. The process, according to claim 1, whereby beta-mercaptopropionylamidoacetic acid is prepared from a bromopropyl-halogenide and glycine by reacting their cond_nsation product with thiobenzoic acid and alkaline hydrolysing of the beta-benzoilmercaptopropionamidoacetic acid thus obtained.

3. The process, according to claim 1, in which beta-mercaptopropionylamidoacetic acid is prepared from propiothiolactone and ethylic ester of glycine and by alkaline hydrolysis of the ethylic ester of the beta-mercaptopropionylamidoacetic acid thus obtained.

4. The process, according to claim 1, whereby beta-mercaptopropionylamidoacetic acid is prepared from beta-mercaptopropionic acid by oxidixing with $H_2O_2$; beta, beta'-dithio-propionic acid is transformed into the corresponding dihalo-genide; reacting this product with glycine is then done followed by the electrochemical reduction of the beta, beta'-dithiodipropionamidoacetic acid thus obtained.